# Europäisches Patentamt
## European Patent Office
### Office européen des brevets

(11) Veröffentlichungsnummer : **0 011 848**
**B1**

(12)
# EUROPÄISCHE PATENTSCHRIFT

(45) Veröffentlichungstag der Patentschrift :
30.09.81

(51) Int. Cl.³ : **A 61 B   6/02**, H 05 G   1/42,
**A 61 B   6/10**

(21) Anmeldenummer : 79104698.0

(22) Anmeldetag : 26.11.79

(54) Schichtgerät zur Herstellung von Transversalschichtbildern eines Aufnahmeobjektes.

(30) Priorität : 30.11.78 US 965152

(43) Veröffentlichungstag der Anmeldung :
11.06.80 (Patentblatt 80/12)

(45) Bekanntmachung des Hinweises auf die Patenterteilung : 30.09.81 Patentblatt 81/39

(84) Benannte Vertragsstaaten :
DE FR GB

(56) Entgegenhaltungen :
DE - A - 2 627 885
DE - A - 2 628 493
FR - A - 2 073 709
FR - A - 2 237 606
FR - A - 2 270 754
FR - A - 2 296 185
FR - A - 2 313 902

(73) Patentinhaber : SIEMENS AKTIENGESELLSCHAFT
Berlin und München
Postfach 22 02 61
D-8000 München 22 (DE)

(72) Erfinder : Neumann, Leopold
9 Woodpark Circle
Lexington, Massachusetts 02173 (US)

Imprimerie Jouve, 17, rue du Louvre, 75001 Paris, France

## Schichtgerät zur Herstellung von Transversalschichtbildern eines Aufnahmeobjektes

Die Erfindung bezieht sich auf ein Schichtgerät zur Herstellung von Transversalschichtbildern eines Aufnahmeobjektes mit einer Strahlenmeßanordnung, die eine Strahlenquelle, welche ein das Aufnahmeobjekt durchdringendes Strahlenbündel erzeugt, dessen Querschnittsausdehnung senkrecht zur Schichtebene gleich der Schichtstärke ist, sowie einen aus mehreren Detektoren bestehenden Strahlenempfänger enthält, der die Strahlungsintensität hinter dem Objekt ermittelt, sowie mit einer Drehvorrichtung für die Meßanordnung zur Durchstrahlung des Aufnahmeobjektes aus verschiedenen Richtungen und mit einem Meßwertumformer für die Transformation der vom Strahlenempfänger gelieferten Signale in ein Schichtbild.

Bei der Konstruktion von Röntgensystemen zur Erzeugung von Bildern menschlicher Objektes ist einer der wichtigsten Gesichtspunkte, die Strahlendosis zu reduzieren, der der Patient ausgesetzt ist. In einem computerisierten Tomographie-Röntgenabtastsystem der genannten Art ist im allgemeinen eine Röntgenstrahlenquelle und eine Reihe von Strahlungsdetektoren im Strahlenempfänger vorhanden, die beide relativ zu dem abzubildenden Körper bewegbar sind. Während die Röntgenstrahlenquelle und der Strahlenempfänger relativ zum Patienten bewegt werden, wird eine große Zahl von Röntgenprojektionen durchgeführt und die Daten dieser Projektionen werden verarbeitet, um eine Repräsentation der Dichte im Querschnitt des Aufnahmeobjektes zu erhalten.

Bei bekannten Tomographie-Röntgenabtastsystemen wird der Strahlungspegel der Röntgenstrahlenquelle vor dem Beginn eines Aufnahmevorganges gewählt. Die gewählten Aufnahmewerte hängen von den Abmessungen des Patienten und von dem zu untersuchenden Abschnitt des Körpers ab und werden nicht geändert, während die Strahlenmeßanordnung um den Patienten rotiert. Da die Schwächung im Körper für Projektionen unter verschiedenen Winkeln unterschiedlich ist, resultiert aus der Verwendung einer einzigen vorgewählten Aufnahmewertekombination eine höhere Strahlendosis für den Patienten als für die meisten Projektionen erforderlich ist. Weiterhin werden bei den bekannten Systemen die Aufnahmewerte unter Benutzung von Tabellen festgelegt, die auf typischen Größen- und Gewichtscharakteristiken von Patienten beruhen und diese Tabellen ergeben nicht immer für den aktuellen Patienten die optimalen Aufnahmewerte.

In der FR-A 22 96 185 ist bereits ein Schichtgerät beschrieben, beidem die Strahlenquelle für jede Aufnahme individuell derart einstellbar ist, daß der Strahlenempfänger an vorgegebenes minimales Signal erhalt. Bei diesem Schichtgerat besitzt der Strahlenempfänger jedoch nur einen einzigen Detektor. In der DE-A-26 27 885 ist zwar ein Schichtgerät mit einer Vielzahl von Detektoren im Strahlenempfänger beschrieben, bei dem die Detektor-Ausgangssignale mit einem Schwellwert verglichen werden, jedoch erfolgt dieser Vergleich, um fehlerhafte Detektoren im Strahlenempfänger zu identifizieren.

Der vorliegenden Erfindung liegt die Aufgabe zugrunde, ein Schichtgerät der eingangs genannten Art zu schaffen, bei dem die Aufnahmewerte jeder Projektion individuell einstellbar sind, um an einem aus einer Vielzahl von Detektoren bestehenden Strahlenempfänger ein vorgegebenes minimales Signal zu erhalten, das aus einem gewünschten minimalen Signal-Rausch-Verhältnis resultiert. Durch die Wahl des Signal-Rausch-Verhältnisses unter Berücksichtigung des Minimums, das für die Erzeugung eines Bildes einer annehmbaren Qualität erforderlich ist, kann die Strahlungsdosis für den Patienten so klein wie möglich gehalten werden.

Diese Aufgabe ist bei einer Ausführungsform der Erfindung gelöst durch folgende Merkmale :

Mittel zur Einstellung einer Schwelle und zur Erzeugung eines dafür repräsentativen Signales ;

Mittel zur Überwachung des Ausgangssignales einer Vielzahl der Detektoren, um zu erfassen, wenn die überwachten Ausgangssignale die Schwelle überschreiten und um ein für ein solches Überschreiten repräsentatives Zwischensignal zu erzeugen ;

Steuermittel, die mit der Strahlenquelle verbunden sind und auf ein Startsignal ansprechen, um zu veranlassen, daß die Strahlenquelle einen Strahlimpuls beim Auftreten des Startsignales aussendet ; und

Mittel zum Zuführen des Zwischensignales zu den Steuermitteln, wobei die Steuermittel auf das Zwischensignal ansprechen, um die Strahlenquelle zu veranlassen, die Strahlenaussendung zu beenden, wenn die Schwelle von den überwachten Ausgangssignalen überschritten wird, wobei die Überwachungsmittel enthalten :

eine Vielzahl von Komparatoren, von denen jeder zwei Eingangssignale vergleicht und ein Ausgangssignal erzeugt, das repräsentativ für die relativen Größen davon ist, wobei jeder Komparator als ein Eingangssignal eines der Vielzahl von überwachten Detektorausgangssignalen empfängt und als anderes Eingangssignal das Ausgangssignal der die Schwelle festlegenden Mittel, und Gattermittel, die auf die Komparatorausgangssignale ansprechen, um Ausgangssignale zu erzeugen, wenn alle überwachten Detektorausgangssignale die Schwelle überschritten haben, wobei die Ausgangssignale der Gattermittel das genannte Zwischensignal darstellen.

Von der einen Patienten durchsetzenden Strahlung ist diejenige Strahlung, die durch das Zentrum des Patienten verläuft, normalerweise um den größten Wert geschwächt. Indem Signale zentral angeordneter Detektoren, die diese Strahlung empfangen, überwacht werden kann festgestellt werden, wann die Signale dieser Detektoren eine vorbestimmte Schwelle überschreiten und wann eine Projektion beendet werden kann, um ein Bild der gewünschten Qualität zu erzeugen.

Eine andere Ausführungsform, die sowohl für gepulste als auch kontinuierlich arbeitende Systeme anwendbar ist, ist gekennzeichnet durch folgende Merkmale :

Steuermittel, die mit der Strahlenquelle gekuppelt sind, um in Abhängigkeit von einem Eingangssignal die Intensität der von der Strahlenquelle während einer Aufnahme emittierten Strahlung zu steuern ;

Aufnahmemittel, die auf die Ausgangssignale einer Vielzahl der Detektoren im Strahlenempfänger ansprechen, um das Niveau der Strahlung, die von dem Aufnahmeobjekt während jeder Projektion empfangen wird, zu bestimmen und ein dafür repräsentatives Ausgangssignal für jeden der Detektoren zu erzeugen ;

Mittel, die auf die Ausgangssignale der Aufnahmemittel für jede Projektion ansprechen, um die Strahlungsmenge während der nächsten Aufnahme zu bestimmen, und zwar im Hinblick auf die Festlegung eines gewünschten Niveaus empfangener Strahlung von jedem der Detektoren und zur Erzeugung eines Steuersignales, das repräsentativ für das gewünschte Niveau ist, und

Mittel zum Zuführen des Steuersignales als Eingangssignal zu den Steuermitteln.

Dabei werden ausgewählte Detektorausgangssignale nach jeder Projektion mit einem gewünschten Minimalwert verglichen. Die nächste Projektion wird entsprechend den Ergebnissen dieses Vergleiches variiert. Der Projektionswinkel zwischen aufeinanderfolgenden Projektionen ändert sich um einen kleinen Wert in der Größenordnung von einem Winkelgrad oder weniger und die Schwächung der Röntgenstrahlenenergie zwischen aufeinanderfolgenden Projektionen wird sich nur geringfügig ändern. So wird die Bestimmung der richtigen Aufnahmewerte für die nächste Projektion aufgrund der Schwächung, die aus der letzten Projektion bestimmt worden ist, eine Aufnahme ergeben, die für die praktischen Zwecke den optimalen Aufnahmewerten entspricht.

Die Erfindung ist nachfolgend in Verbindung mit den Zeichnungen näher erläutert. Es zeigen :

Figur 1 eine bildliche Darstellung eines Schichtgerätes, in dem die Erfindung angewandt. werden kann,

Figur 2 eine schematische Seitenansicht des Abtastsystems bei dem Gerät gemäß Figur 1,

Figur 3 ein Blockschaltbild zur Erläuterung einer Ausführungsform der Erfindung zur Benutzung mit gepulsten Abtastsystemen, und

Figur 4 ein Blockschaltbild einer anderen Ausführungsform der Erfindung.

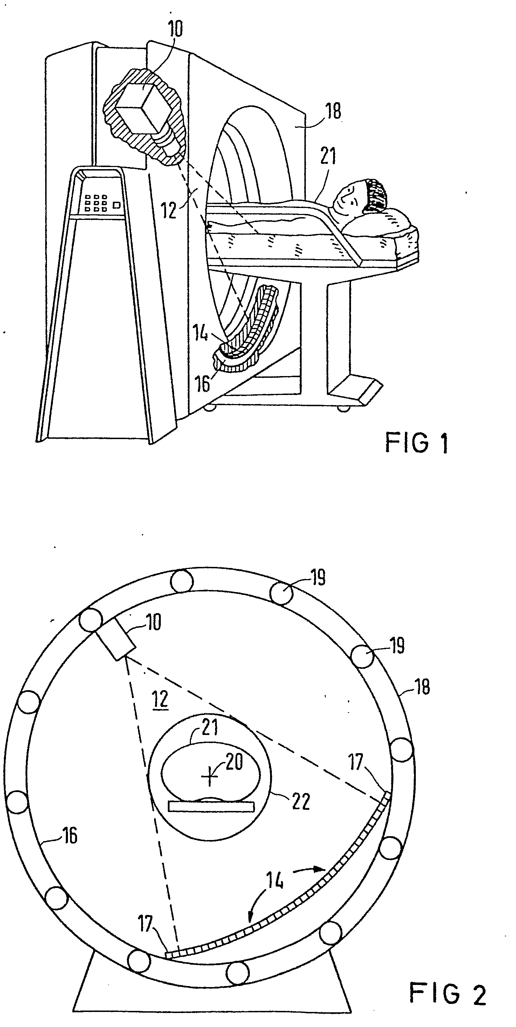

In den Figuren 1 und 2 ist ein Schichtgerät mit einem fächerförmigen Strahlenbündel gezeigt, bei dem die Erfindung anwendbar ist. Die Erfindung ist aber in gleicher Weise bei Computertomographen anwendbar, die nicht mit Fächerstrahlen arbeiten, wie Parallelstrahl-Computertomographen und Systeme, die andere als Röntgenstrahlung verwenden. Das beschriebene System stellt nur ein Beispiel dar und ist nicht als Beschränkung der vorliegenden Erfindung aufzufassen.

Das Schichtgerät weist eine Röntgenröhre 10 auf, die ein fächerförmiges Strahlenbündel 12 aussendet, das durch einen Patienten 21, der abgetastet wird, dringt und auf einem Strahlenempfänger 14 auftrifft, der aus einer Reihe vieler Detektoren besteht, die Ausgangssignale erzeugen, die der Intensität der empfangenen Strahlung proportional sind. Die Abmessung des Röntgenstrahlenbündels 12 senkrecht zur Ebene der untersuchten Schicht ist gleich der Schichtstärke. Die Röntgenstrahlenquelle 10 und der Strahlenempfänger 14 sind auf einem inneren Ring 16 einer Abtasteinheit angeordnet, so daß die Strahlenquelle 10 und der Strahlenempfänger 14 in einer festen Beziehung zueinander gehalten werden. Der innere Ring 16 kann in einem äußeren Ring 18 gedreht werden, und zwar mittels Rollen 19, so daß der innere Ring 16 die Röntgenstrahlenquelle 10 und den Strahlenempfänger 14 um die Achse 20, die das Zentrum des Ringes 16 darstellt, und die senkrecht zur Ebene des fächerförmigen Strahlenbündels 12 verläuft, dreht.

Während die Röntgenstrahlenquelle 10 und der Strahlenempfänger 14 um den Patienten 21 rotieren, wird eine Vielzahl von Röntgenaufnahmen, sog. Projektionen des Patienten, unter verschiedenen Winkeln durchgeführt. Auf diese Weise durchdringt das Strahlenbündel 12 einen kreisförmigen Bereich, der durch die Linie 22 in der Figur 2 festgelegt ist, während der innere Ring 16 eine vollständige Umdrehung, einen sog. Scan, ausführt. Der Patient 21 ist in dem kreisförmigen Bereich 22 angeordnet und die Röntgenstrahlen, die durch den Patienten dringen, treffen auf dem Strahlenempfänger 14 auf. Der Strahlenempfänger 14 besitzt Überwachungsdetektoren 17, die an einem oder beiden Enden angeordnet sind und ungeschwächte Röntgenstrahlen empfangen, die außerhalb des ringförmigen Bereiches 22 passieren. Auf diese Weise wird durch die Röntgenstrahlen, die auf den Überwachungsdetektoren 17 auftreffen, ein Signal für die Röntgenstrahlungsintensität der Röntgenstrahlenquelle 10 bei jeder Projektion erzeugt, weil diese Röntgenstrahlen nicht durch den Patienten 21 geschwächt werden.

Der Durchstrahlungswinkel der Röntgenstrahlenquelle 10, der den Bereich 22 festlegt, wird der Projektionswinkel genannt. Indem Daten des Strahlenempfängers 14 unter einer Vielzahl von verschiedenen Projektionswinkeln abgeleitet werden, wird ein tomographischer Scan eines Patienten oder eines anderen Objektes im kreisförmigen Bereich 22 vorgenommen. Durch geeignete Verarbeitung der Daten des Strahlenempfängers 14 in einem Meßwertumformer kann ein Bild der Dichteverteilung des Patienten in dem untersuchten Querschnitt innerhalb des Strahlenbündels 12 erzeugt werden. Ein typisches Tomographiesystem, in dem die vorliegende Erfindung angewendet werden kann, ist in der US-PS 41 35 247 beschrieben.

Bei Schichtgeräten der beschriebenen Art besteht der Wunsch, die Strahlendosis, der der Patient unterworfen wird, so klein wie möglich zu halten. Aufgrund der Tatsache, daß ein tomographischer Scan eine große Zahl von Projektionen erfordert, nämlich üblicherweise Hunderte von Projektionen, ist dieses Problem hier größer als bei konventionellen Röntgensystemen.

Während die Röntgenstrahlenquelle 10 und der Strahlenempfänger 14 um den Patienten 1 rotieren, ändert sich die Länge der Strahlenwege und damit auch die Schwächung der Röntgenstrahlung in einem weiten Bereich. Zum Beispiel ist im Hinblick auf die Figur 2 in dem Fall, in dem die Röntgenstrahlenquelle 10 über dem Patienten 21 liegt, die Dicke des Körperbereiches, durch den die Röntgenstrahlen passieren und von dem sie geschwächt werden, wesentlich kleiner als in dem Fall, in dem die Röntgenstrahlenquelle 10 und der Strahlenempfänger 14 horizontal orientiert sind.

In bekannten Systemen mit fest eingestellten Aufnahmewerten wird die Strahlungsintensität so gewählt, daß am Strahlenempfänger 14 bei der höchsten Schwächung des Strahlenbündels 12 noch ausreichende Strahlenpegel erreicht werden. Dieser Fall würde in Figur 2 auftreten, wenn die Röntgenstrahlenquelle 10 und der Strahlenempfänger 14 horizontal orientiert wären. Für andere Projektionswinkel ist die Schwächung des Röntgenstrahlenbündels 12 durch den Patienten 21 geringer und die Aufnahmewerte und damit die Strahlenbelastung des Patienten ist höher als notwendig.

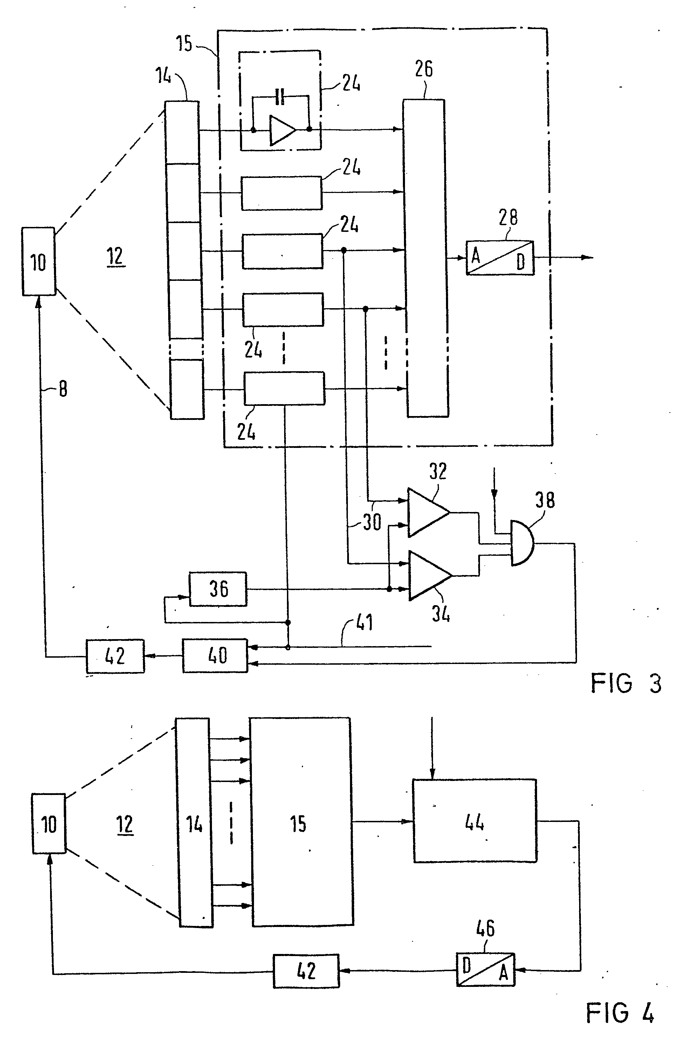

Entsprechend der vorliegenden Erfindung werden die Aufnahmewerte für die Röntgenstrahlenquelle 10 auf einen Optimalwert für jede Projektion während eines Scan eingestellt, so daß die Strahlenbelastung des Patienten 21 ohne Beeinflussung der Bildqualität reduziert wird. In der Figur 3 ist eine bevorzugte Ausführungsform der vorliegenden Erfindung gezeigt, die insbesondere für die Benutzung mit einem Röntgenstrahlenscanner geeignet ist, bei dem während eines Aufnahmevorganges die Röntgenstrahlenquelle 10 gepulst und die Pulsbreite der Röntgenstrahlenemission der Röntgenstrahlenquelle 10 verändert wird.

Die Schaltung wirkt in der folgenden Weise. Entsprechend einem Puls auf der Leitung 8 zur Röntgenstrahlenquelle 10 erzeugt die Röntgenstrahlenquelle 10 einen Strahlungsimpuls, der auf dem Strahlenempfänger 14 auftrifft und eine Dauer hat, die im wesentlichen gleich der Breite des Pulses auf der Leitung 8 ist. Der Patient ist in der Figur 3 nicht im Strahlenbündel 12 dargestellt.

Der Strahlenempfänger 14 enthält eine Vielzahl individueller Detektoren, die die Röntgenstrahlung empfangen und Ausgangssignale erzeugen, die dem Niveau der empfangenen Strahlung entsprechen. Die Signale des Strahlenempfängers 14 werden einem Datenerfassungssystem 15 zugeführt, das die Analogsignale niedrigen Niveaus der Detektoren in digitalisierte Signale umwandelt, die dem tomographischen Prozessor zugeführt werden, der das endgültige Bild erzeugt. Das Ausgangssignal jedes Detektors des Strahlenempfängers 14 wird einem entsprechenden rückstellbaren Integrator 24 zugeführt. Die Integratoren 24 werden vor jeder Projektion zurückgestellt. Das Ausgangssignal jedes der Detektoren im Strahlenempfänger 14 wird in dem entsprechenden Integrator 24 während einer Projektion, d.h. während eines Pulses der Röntgenstrahlenquelle 10 integriert, um ein Ausgangssignal zu erzeugen, das der empfangenen Röntgenstrahlungsintensität entspricht. Die Ausgangssignale jedes der Integratoren 24 werden einem Analogmultiplexer 26 zugeführt. Der Multiplexer 26 führt aufeinanderfolgend jedes der Ausgangssignale der Integratoren 24 einem Analog-Digital-Konverter 28 zu, welcher eine digitale Repräsentation jedes der Ausgangssignale der Integratoren 24 erzeugt. Die digitalen Signale werden dann in einem bekannten Tomographieprozessor verarbeitet, um das endgültige Bild des untersuchten Querschnittes zu erzeugen.

Die Steuerung der Aufnahmewerte erfolgt gemäß der vorliegenden Erfindung in der folgenden Weise. Die Ausgangssignale mehrerer der Integratoren 24 werden entsprechenden Komparatoren zugeführt, welche festlegen, ob das entsprechende Ausgangssignal der Integratoren 24 eine vorbestimmte Schwelle überschritten hat. In der Figur 3 werden die Ausgangssignale zweier Integratoren 24 über Leitungen 30 den ersten Eingängen von Komparatoren 32 und 34 zugeführt. Die zweiten Eingänge der Komparatoren 32 und 34 werden von einem Schwellengenerator 36 gespeist. Im allgemeinen werden die Ausgangssignale von Integratoren 24 benutzt, die Detektoren zugeordnet sind, die zentral im Strahlenempfänger liegen, da diese Detektoren normalerweise Strahlung mit dem niedrigsten Niveau empfangen. Der Schwellwert des Schwellengenerators 36 wird so gewählt, daß er dem kleinst möglichen Signal der Integratoren 24 entspricht, das das gewünschte Signal-Rausch-Verhältnis ergibt. Die Ausgangssignale der Komparatoren 32 und 34 werden einem UND-Gatter 38 zugeführt. Als Antwort auf ein hohes Niveau beider Komparatoren 32 und 34, das anzeigt, daß die entsprechenden Ausgangssignale der Integratoren 24 auf den Leitungen 30 beide den Schwellwert des Schwellengenerators 36 überschritten haben, stellt sich das Ausgangssignal des UND-Gatters 38 auf ein hohes Niveau ein und zeigt an, daß eine Röntgenaufnahme beendet verden sollte.

Ein Pulsbreitenkreis 40 empfängt einen Startimpuls auf einer Leitung 41, der jede Röntgenaufnahme während jeder Projektion auslöst. Der Startimpuls kann auch dazu benutzt werden, die Integratoren 24 zurückzustellen. Das Ausgangssignal des UND-Gatters 38, das anzeigt, wenn eine Aufnahme beendet werden soll, wird auch dem Pulsbreitenkreis 40 zugeführt. Als Antwort auf einen Startimpuls erzeugt der Pulsbreitenkreis 40 ein Signal für einen Röntgensteuerkreis 42, der den Aufnahmebeginn festlegt. Wenn das gewünschte Niveau erreicht worden ist, erzeugt das UND-Gatter 38 ein Signal, das dem Pulsbreitenkreis 40 zugeführt wird. Als Antwort auf dieses Signal bewirkt der Pulsbreitenkreis 40 über den Röntgensteuerkreis 42 eine Ausschaltung der Röntgenstrahlenquelle 10 zur Beendigung einer Aufnahme. Auf diese Weise wird die Strahlendosis, der ein Patient ausgesetzt wird, auf einem Minimum gehalten, während eine annehmbare Bildqualität erzielt wird.

Nach einem Steuersignal für die Ausschaltung der Röntgenstrahlenquelle 10 vergeht ein Zeitintervall bis die Röntgenstrahlenquelle 10 auch tatsächlich ausgeschaltet ist. Zur Kompensation dieses Zeitintervalles ist es möglich, die Schwelle im Schwellengenerator 36 entsprechend zu reduzieren, so daß die Strahlung der Röntgenstrahlenquelle 10 in der vorgesehenen Zeit beendet wird. Der Schwellengenerator 36 kann in bekannter Technik ausgeführt sein, um eine Schwellencharakteristik zu bilden, die die Verzögerungscharakteristiken der Röntgenstrahlenquelle und der zugeordneten Steuerelektronik berücksichtigt.

Eine andere Ausführungsform ist in der Figur 4 dargestellt. Hier wird die Dauer von Aufnahmen entsprechend dem Strahlungsniveau bestimmt, das der Strahlenempfänger während der vorhergehenden Aufnahme empfangen hat. In der Figur 4 erzeugt wieder die Röntgenstrahlenquelle 10 ein Strahlenbündel 12, das auf einem Strahlenempfänger 14, wie oben beschrieben, auftrifft. Die Ausgangssignale der Detektoren im Strahlenempfänger 14 werden einem Datenerfassungssystem 15 zugeführt, das dem Datenerfassungssystem 15 der Figur 3 entspricht, und die Ausgangsdaten dieses Datenerfassungssystems 15 werden einem tomographischen Prozessor 44 zugeführt, der das endgültige Bild, das den untersuchten Querschnittsbereich repräsentiert, erzeugt. Nach dem Abschluß jeder Projektion oder Aufnahme wird die Dauer der nächsten Aufnahme entsprechend dem Niveau bestimmt, das vom Strahlenempfänger 14 während der vorhergehenden Aufnahme empfangen worden ist. Die Strahlungsintensität wird bestimmt, indem die Ausgangssignale mehrerer Detektoren des Strahlenempfängers 14 überwacht werden, um ein minimales Signalniveau zu erreichen, das um einen vorbestimmten Betrag größer ist als das bekannte Rauschniveau, so daß ein gewünschtes Signal-Rausch-Verhältnis erzielt wird.

Die Intensität der nächsten Aufnahme kann durch den Prozessor 44 festgelegt werden, der Daten empfängt, die das Schwellenkriterium repräsentieren, das erforderlich ist, um das gewünschte Signal-Rausch-Verhältnis zu erreichen. Der Prozessor 44 erzeugt nach jeder Projektion ein digitales Signal, das repräsentativ für das Strahlungsniveau der nächsten Projektion ist und dieses digitale Signal wird einem Digital-Analog-Wandler 46 zugeführt, dessen Ausgang ein Analogsignal ist, das einem Röntgensteuerkreis 42 zugeführt wird. Der Röntgensteuerkreis 42 ist mit der Röntgenstrahlenquelle 10 verbunden und bewirkt die Erzeugung einer gewünschten Aufnahme durch die Röntgenstrahlenquelle 10 während der nächsten Projektion. Der Prozessor 44 ist ein Prozessor auf Computerbasis, in dem die gewünschten Kontrollfunktionen durch geeignete Software spezifiziert sind. Die Bestimmung einer Aufnahme kann auch durch spezielle Analog- oder Digitalkreise erfolgen, die mit dem Datenerfassungssystem 15 zusammenarbeiten oder auf andere Weise im Tomographiesystem angeordnet sind. Die Berechnung des Aufnahmepegels für jede Projektion auf der Basis des Strahlenpegels der vorhergehenden Projektion verursacht nur einen kleinen Fehler, da die Bewegung der Röntgenstrahlenquelle 10 und des Strahlenempfängers 14 zwischen zwei Projektionen gering ist, im allgemeinen 1 Winkelgrad oder weniger. Auf diese Weise ändert sich das Niveau der empfangenen Strahlung nicht stark zwischen einer Projektion und der nächsten.

Der Röntgensteuerkreis 42 verändert einen oder mehrere Parameter der Röntgenstrahlenquelle 10 um die gewünschte Strahlenqualität für eine Aufnahme zu erzielen. In einem fortlaufend arbeitenden System ist der am besten geeignete Parameter der Röntgenröhrenstrom, während in einem gepulsten Röntgensystem die Pulsbreite am leichtesten variiert werden kann. Es können aber auch andere Parameter variiert werden, um die gewünschte Strahlungsqualität für eine Aufnahme zu erzielen. Die Festlegung des nächsten Aufnahmeniveaus kann auch auf allen Detektorausgangssignalen oder einer Auswahl von Detektorausgangssignalen basieren, deren Detektoren nahe dem Zentrum des Strahlenempfängers 14 liegen, das normalerweise das minimale Signal empfängt.

Vorstehend ist ein neues System zur Steuerung der Röntgenaufnahmen in einem tomographischen Scanner beschrieben, um die Strahlendosis, die auf einen Patienten einwirkt, auf ein Minimum zu bringen, während eine gewünschte Bildqualität erreicht wird. Modifikationen der bevorzugten Ausführungsformen der Erfindung, die oben beschrieben sind, können vom Fachmann durchgeführt werden, ohne den Rahmen der Erfindung zu verlassen.

## Ansprüche

1. Schichtgerät zur Herstellung von Transversalschichtbildern eines Aufnahmeobjektes mit einer Strahlenmeßanordnung, die eine Strahlenquelle (10), welche ein das Aufnahmeobjekt (21) durchdringendes Strahlenbündel (12) erzeugt, dessen Querschnittsausdehnung senkrecht zur Schichtebene gleich der Schichtstärke ist, sowie einen aus mehreren Detektoren bestehenden Strahlenempfänger (14) enthält, der die Strahlungsintensität hinter dem Objekt (21) ermittelt, sowie mit einer Drehvorrichtung (16, 18, 19) für die Meßanordnung zur Durchstrahlung des Aufnahmeobjektes (21) aus verschiedenen Richtungen und mit einem Meßwertumformer für die Transformation der vom Strahlenempfänger (14) gelieferten Signale in ein Schichtbild, das durch folgende Merkmale gekennzeichnet ist :

Mittel (36) zur Einstellung einer Schwelle und zur Erzeugung eines dafür repräsentativen Signales ;

Mittel (32, 34, 38) zur Überwachung des Ausgangssignales einer Vielzahl der Detektoren, um zu erfassen, wenn die überwachten Ausgangssignale die Schwelle überschreiten und um ein für 'ein solches Überschreiten repräsentatives Zwischensignal zu erzeugen ;

Steuermittel (40, 42), die mit der Strahlenquelle (10) verbunden sind und auf ein Startsignal ansprechen, um zu veranlassen, daß die Strahlenquelle (10) einen Strahlimpuls beim Auftreten des Startsignales aussendet ; und Mittel zum Zuführen des Zwischensignales zu den Steuermitteln (40, 42), wobei die Steuermittel (40, 42) auf das Zwischensignal ansprechen, um die Strahlenquelle (10) zu veranlassen, die Strahlenaussendung zu beenden, wenn die Schwelle von den überwachten Ausgangssignalen überschritten wird, wobei die Überwachungsmittel enthalten :

eine Vielzahl von Komparatoren (32, 34), von denen jeder zwei Eingangssignale vergleicht und ein Ausgangssignal erzeugt, das repräsentativ für die relativen Größen davon ist, wobei jeder Komparator (32, 34) als ein Eingangssignal eines der Vielzahl von überwachten Detektorausgangssignalen empfängt und als anderes Eingangssignal das Ausgangssignal der die Schwelle festlegenden Mittel (36), und

Gattermittel (38), die auf die Komparatorausgangssignale ansprechen, um Ausgangssignale zu erzeugen, wenn alle überwachten Detektorausgangssignale die Schwelle überschritten haben, wobei die Ausgangssignale der Gattermittel (38) das genannte Zwischensignal darstellen.

2. Schichtgerät nach Anspruch 1, dadurch gekennzeichnet, daß mindestens einer der Detektoren, die von den Überwachungsmitteln (32, 34, 38) überwacht werden, zentral im Strahlenempfänger (14) angeordnet ist.

3. Schichtgerät zur Herstellung von Transversalschichtbildern eines Aufnahmeobjektes mit einer Strahlenmeßanordnung, die eine Strahlenquelle (10), welche ein das Aufnahmeobjekt (21) durchdringendes Strahlenbündel (12) erzeugt, dessen Querschnittsausdehnung senkrecht zur Schichtebene gleich der Schichtstärke ist, sowie einen aus mehreren Detektoren bestehenden Strahlenempfänger (14) enthält, der die Strahlungsintensität hinter dem Objekt (21) ermittelt, sowie mit einer Drehvorrichtung (16, 18, 19) für die Meßanordnung zur Durchstrahlung des Aufnahmeobjektes (21) aus verschiedenen Richtungen und mit einem Meßwertumformer für die Transformation der vom Strahlenempfänger (14) gelieferten Signale in ein Schichtbild, gekennzeichnet durch folgende Merkmale :

Steuermittel (42), die mit der Strahlenquelle (10) gekuppelt sind, um in Abhängigkeit von einem Eingangssignal die Intensität der von der Strahlenquelle (10) während einer Aufnahme emittierten Strahlung zu steuern ;

Aufnahmemittel (15), die auf die Ausgangssignale einer Vielzahl der Detektoren im Strahlenempfänger (14) ansprechen, um das Niveau der Strahlung, die von dem Aufnahmeobjekt (21) während jeder Projektion empfangen wird, zu bestimmen und ein dafür repräsentatives Ausgangssignal für jeden der Detektoren zu erzeugen ;

Mittel (44), die auf die Ausgangssignale der Aufnahmemittel (15) für jede Projektion ansprechen, um die Strahlungsmenge während der nächsten Aufnahme zu bestimmen, und zwar im Hinblick auf die Festlegung eines gewünschten Niveaus empfangener Strahlung von jedem der Detektoren und zur Erzeugung eines Steuersignales, das repräsentativ für das gewünschte Niveau ist ; und Mittel (46) zum Zuführen des Steuersignales als Eingangssignal zu den Steuermitteln (42).

4. Schichtgerät nach Anspruch 3, dadurch gekennzeichnet, daß die Steuermittel (42) Mittel zur Regelung eines Stromes beinhalten, der der Strahlenquelle (10) zugeführt wird, um die Strahlungsqualität zu steuern.

5. Schichtgerät nach Anspruch 3, dadurch gekennzeichnet, daß die Strahlenquelle (10) während jeder Aufnahme einen Strahlungsimpuls aussendet, und daß die Steuermittel (42) Mittel zur Steuerung der Länge des Strahlungsimpulses der Strahlenquelle (10) während jeder Aufnahme beinhalten.

6. Schichtgerät nach Anspruch 3, dadurch gekennzeichnet, daß es von dem Typ ist, bei dem die Strahlenquelle (10) und der Strahlenempfänger (14) um das Aufnahmeobjekt (22) rotieren, um eine Serie von Aufnahmen zu erzeugen.

7. Schichtgerät nach Anspruch 6, dadurch gekennzeichnet, daß die Strahlenquelle (10) ein fächerförmiges Strahlenbündel (12) aussendet.

**Claims**

1. Apparatus for the production of a transversal tomograph of an object to be recorded, comprising a beam measuring arrangement having a beam source (10) producing a beam (12) which penetrates the object (21) for recording and whose cross-sectional dimension at right angles to the plane of recording is equal to the thickness thereof, and comprising a beam receiver (14) having a plurality of detectors for determining the radiation intensity behind the object (21), and further comprising a rotating device (16, 18, 19) for the measuring arrangement permitting irradiation of the object (21) from various directions, and a measured value converter for the transformation of the signals supplied by the beam receiver (14) into a tomograph, characterised by the following features :

means (36) for setting a threshold level and producing a signal representative thereof ;

means (32, 34, 38) for monitoring the output signal of a plurality of the detectors in order to detect when the monitored output signals exceed the threshold level and thereupon produce an intermediate signal representating such ;

control means (40, 42) connected to the beam source (10) which respond to a start signal causing the beam source (10) to emit a pulse ; and means for supplying the intermediate signal to the control means (40, 42) which respond to the intermediate signal to cause the beam source (10) to terminate the beam transmission when the threshold level is exceeded by the monitored output signals, where the monitoring means contain :

a plurality of comparators (32, 34), each of which compares two input signals and produces an output signal representative of the relative values and each comparator (32, 34) receives as one input signal one of the plurality of monitored detector output signals, and as another input signal receives the output signal from the means (36) which determine the threshold level, and

gate means (38) which respond to the comparator output signals in order to produce output signals if all the monitored detector output signals have exceeded the threshold level, where the output signals of the gate means (38) represent the aforementioned intermediate signal.

2. Apparatus as claimed in Claim 1 characterised in that at least one of the detectors which are monitored by the monitoring means (32, 34, 38) is arranged centrally in the beam receiver (14).

3. Apparatus for the production of transversal tomographs of an object to be recorded, comprising a beam measuring arrangement which contains a beam source (10) producing a beam (12) which penetrates the object (21) to be recorded and whose cross-sectional dimension at right angles to the plane of recording is equal to the thickness thereof, and further contains a beam receiver (14) which consists of a plurality of detectors which detect the radiation intensity behind the object (21), and comprising a rotation device (16, 18, 19) for the measuring arrangement enabling irradiation of the object (21) to be recorded from various directions, and further comprising a measured value converter for the transformation of the signal supplied by the beam receiver (14) into a tomograph, characterised by the following features :

control means (42) which are coupled to the beam source (10) in order to control the intensity of the radiation emitted by the beam source (10) during a recording process in dependence upon an input signal ;

recording means (15) which respond to the output signals of a plurality of the detectors in the beam receiver (14) in order to determine the level of the radiation which is received from the object (21) to be recorded during each projection and in order to produce an output signal representative thereof for each of the detectors ;

means (44) which respond to the output signals of the recording means (45) for each projection to determine the quantity of radiation during the next recording by detecting a desired level of received radiation from each of the detectors and producing a control signal representative of the desired level, and means (46) for supplying the control signal as an input signal to the control means (42).

4. Apparatus as claimed in Claim 3, characterised in that the control means (42) contain means for regulating a current which is conducted to the beam source (10) in order to control the radiation quality.

5. Apparatus as claimed in Claim 3, characterised in that the beam source (10) transmits a radiation pulse during each recording process, and that the control means (42) contain means for controlling the length of the radiation pulse of the radiation source (10) during each recording process.

6. Apparatus as claimed in Claim 3, characterised in that the beam source (10) and the beam receiver (14) rotate around the object (22) which is to be recorded in order to produce a series of photographs.

7. Apparatus as claimed in Claim 6, characterised in that the beam source (10) transmits a fan-shaped beam (12).

**Revendications**

1. Appareil d'obtention de tomographies transversales d'un organe absorbant, comprenant un montage de mesure de rayonnement, à source (10) qui donne un faisceau (12) de rayonnement, traversant l'organe (21) absorbant et ayant une étendue transversale perpendiculairement au plan de coupe égale à l'épaisseur de la coupe, et à récepteur (14) de rayonnement constitué de plusieurs détecteurs et donnant l'intensité du rayonnement derrière l'organe (21) absorbant, ainsi qu'un dispositif (16, 18, 19) pour faire tourner le montage de mesure, en vue d'irradier l'organe (21) absorbant, suivant des directions diverses, et qu'un transducteur de mesure, destiné à transformer les signaux fournis par le récepteur (14) en une tomographie, caractérisé par les dispositions suivantes :

des moyens (36) de réglage d'un seuil et d'obtention d'un signal représentatif de celui-ci ;

des moyens (32, 34, 38) de surveillance du signal de sortie d'un grand nombre des détecteurs afin de détecter si le signal de sortie surveillé dépasse le seuil et afin d'élaborer un signal intermédiaire représentatif d'un tel dépassement ;

des moyens (40, 42) de commande qui sont reliés à la source (10) de rayonnement et sont sensibles à un signal de mise en marche afin de faire en sorte que la source (10) de rayonnement émette une impulsion de rayonnement lorsque se produit le signal de mise en marche ; et

des moyens d'acheminement du signal intermédiaire aux moyens (40, 42) de commande, ceux-ci étant sensibles au signal intermédiaire de manière à faire en sorte que la source (10) de rayonnement cesse d'émettre un rayonnement si le seuil est dépassé par les signaux de sortie surveillés, les moyens de surveillance comprenant : un grand nombre de comparateurs (32, 34) dont chacun compare deux signaux d'entrée et élabore un signal de sortie représentatifs des valeurs relatives, chaque comparateur (32, 34) recevant comme signal d'entrée l'un des signaux de sortie du grand nombre de détecteurs surveillés, et, comme autre signal d'entrée, le signal de sortie des moyens (36) déterminant le seuil, et

des moyens (38) à porte qui sont sensibles aux signaux de sortie du comparateur afin d'élaborer des signaux de sortie quand tous les signaux de sortie des détecteurs surveillés ont dépassé le seuil, les signaux de sortie des moyens (38) à portes représentant ledit signal intermédiaire.

2. Appareil suivant la revendication 1, caractérisé en ce qu'au moins l'un des détecteurs qui est surveillé par les moyens (32, 34, 38) de surveillance est disposé au centre du récepteur (14) de rayonnement.

3. Appareil d'obtention de tomographies transversales d'un organe absorbant, comprenant un montage de mesure de rayonnement, à source (10) qui donne un faisceau (12) de rayonnement traversant l'organe (21) absorbant et ayant une étendue transversale perpendiculairement au plan de coupe égale à l'épaisseur de la coupe, et à récepteur (14) de rayonnement constitué de plusieurs détecteurs et donnant l'intensité du rayonnement derrière l'organe (21) absorbant, ainsi qu'un dispositif (16, 18, 19) pour faire tourner le montage de mesure, en vue d'irradier l'organe (21) absorbant, suivant des directions diverses, et qu'un transducteur de mesure, destiné à transformer les signaux fournis par le récepteur (14) en une tomographie, caractérisé par les dispositions suivantes :

des moyens (42) de commande qui sont couplés à la source (10) de rayonnement afin de commander l'intensité du rayonnement émis par la source (10) pendant une prise de vue, en fonction d'un signal d'entrée ;

des moyens (15) d'absorption qui sont sensibles aux signaux de sortie d'un grand nombre de détecteurs du récepteur (14) de rayonnement, afin de déterminer le niveau de rayonnement qui est reçu par l'organe (21) absorbant pendant chaque projection et d'en élaborer un signal de sortie représentatif pour chaque détecteur ;

des moyens (44) qui sont sensibles pour chaque projection aux signaux de sortie des moyens (45) d'absorption afin de déterminer la quantité de rayonnement pendant la prise de vue immédiatement suivante, et cela compte tenu de la fixation d'un niveau le plus souhaité de rayonnement reçu par chacun des détecteurs, et afin d'élaborer un signal de commande représentatif du niveau le plus souhaité ; et des moyens (46) d'acheminement du signal de commande à titre de signal d'entrée aux moyens (42) de commande.

4. Appareil suivant la revendication 3, caractérisé en ce que les moyens (42) de commande comprennent des moyens de régulation d'un courant, qui est envoyé à la source (10) de rayonnement afin de commander la qualité du rayonnement.

5. Appareil suivant la revendication 3, caractérisé en ce que la source (10) de rayonnement émet une impulsion de rayonnement pendant chaque prise de vue, et les moyens (42) de commande comprennent des moyens de commande de la longueur de l'impulsion de rayonnement émise par la source (10) pendant chaque prise de vue.

6. Appareil suivant la revendication 3, caractérisé en ce qu'il est du type suivant lequel la source (10) de rayonnement et le récepteur (14) de rayonnement tournent autour de l'organe (21) absorbant afin d'obtenir une série de prises de vues.

7. Appareil suivant la revendication 6, caractérisé en ce que la source (10) de rayonnement émet un faisceau (12) de rayonnement en forme d'éventail.

FIG 1

FIG 2

2/2

FIG 3

FIG 4